(19) Europäisches Patentamt / European Patent Office / Office européen des brevets

(11) **EP 1 335 663 B1**

(12) **FASCICULE DE BREVET EUROPEEN**

(45) Date de publication et mention
de la délivrance du brevet:
**02.12.2009 Bulletin 2009/49**

(51) Int Cl.:
***A61B 3/16*** *(2006.01)*

(21) Numéro de dépôt: **01978517.9**

(22) Date de dépôt: **09.10.2001**

(86) Numéro de dépôt international:
**PCT/FR2001/003108**

(87) Numéro de publication internationale:
**WO 2002/030274 (18.04.2002 Gazette 2002/16)**

(54) **PROCEDE ET DISPOSITIF DE DETECTION DES MODES PROPRES DE VIBRATION D'UN OEIL, PAR INTERFEROMETRIE LASER, ET LEUR APPLICATION A LA MESURE DE LA PRESSION**

VERFAHREN UND GERÄT ZUR MESSUNG DER EIGENMODEN VON VIBRATIONEN EINES AUGES, MIT EINEM LASERINTERFEROMETER UND DESSEN ANWENDUNG BEI DER MESSUNG DES AUGENINNENDRUCKES

PROCEDE ET DISPOSITIEF DE DETECTION DES MODES PORPRES DE VIBRATION D'UN OEIL, PAR INTERFEROMETRIE LASER, ET LEUR APPLICATION A LA MESURE DE LA PRESSION INTRA-OCULAIRE

(84) Etats contractants désignés:
**AT BE CH CY DE DK ES FI FR GB GR IE IT LI LU MC NL PT SE TR**

(30) Priorité: **10.10.2000 FR 0012957**

(43) Date de publication de la demande:
**20.08.2003 Bulletin 2003/34**

(73) Titulaires:
- **CENTRE HOSPITALIER REGIONAL ET UNIVERSITAIRE DE LILLE**
  **59037 Lille Cédex (FR)**
- **UNIVERSITE DES SCIENCES ET TECHNOLOGIES DE LILLE**
  **59655 Villeneuve-d'Ascq Cedex (FR)**
- **UNIVERSITE DU DROIT ET DE LA SANTE DE LILLE**
  **59800 Lille (FR)**
- **IRIS Pharma**
  **06610 La Gaude (FR)**

(72) Inventeurs:
- **ZEMMOURI, Jaouad**
  **F-59510 Hem (FR)**
- **DUBOIS, Patrick**
  **F-59800 Lille (FR)**
- **ELENA, Pierre-Paul**
  **F-06300 Nice (FR)**
- **ROULAND, Jean-François**
  **F-59800 Lille (FR)**
- **DEBUT, Alexis**
  **F-59000 Lille (FR)**

(74) Mandataire: **Hennion, Jean-Claude**
**Cabinet Beau de Loménie**
**Agence de Lille**
**Immeuble Eurocentre (Euralille)**
**179, boulevard de Turin**
**59777 Lille (FR)**

(56) Documents cités:
**WO-A-96/32054     DE-A- 3 201 801**
**DE-A- 19 647 114    US-A- 5 148 807**

**Description**

La présente invention concerne la détection des modes propres de vibration d'un oeil. Elle trouve préférentiellement, mais non exclusivement, son application à la mesure de la pression intra-oculaire (PIO).

Dans le domaine de l'ophtalmologie, la mesure de la pression intra-oculaire est utilisée par exemple pour réaliser le diagnostic de certaines pathologies ophtalmologiques, dont la principale est le glaucome de l'oeil.

Il existe à ce jour différents dispositifs de mesure de la pression intra-oculaire, qui peuvent être classés en deux catégories principales : les tonomètres, et les dispositifs de mesure par interférométrie laser. Parmi les tonomètres avec contact, on trouve essentiellement les tonomètres par indentation tel que par exemple le tonomètre de Schiotz, et les tonomètres par aplanation, dont le plus répandu est le tonomètre de Goldman.

Les tonomètres par indentation mettent en oeuvre un piston qui est utilisé pour déformer la paroi oculaire, en s'enfonçant dans la cornée, la mesure de PIO étant réalisée en mesurant la longueur de déplacement du piston. L'inconvénient majeur de la tonométrie par indentation est qu'elle oblige à tenir compte de la rigidité de la paroi oculaire, ce qui entraîne une variabilité de la mesure d'un oeil à l'autre. Pour cette raison, on peut considérer que cette technique de mesure n'est plus utilisée à ce jour.

Les tonomètres par aplanation utilise le principe connu selon lequel la pression qui règne à l'intérieur d'une chambre de pression sphérique tel que l'oeil, est en relation avec la force qui est capable d'aplanir une certaine surface de cette sphère.

Plus particulièrement, parmi les tonomètres par aplanation, le tonomètre de Goldman est composé d'un cône d'aplanissement en plastique contenant un biprisme qui transforme l'image ronde de la cornée aplanie en deux demi cercles qui coïncident au moment de l'aplanation de la cornée. Ce cône d'aplanissement est relié par une tige à un système qui génère, grâce à un ressort étalonné, la force nécessaire pour l'aplanation et qui convertit cette force en millimètres de mercure (mm-Hg). Le principal inconvénient de ce tonomètre est que sa mise en oeuvre est traumatisante pour l'oeil et douloureuse, puisque l'on exerce sur la cornée une force mécanique de déformation. Il en résulte que la mesure répétée sur un même oeil peut aboutir à une lésion de l'épithélium cornéen, et cette méthode doit en pratique nécessairement être mise en oeuvre par un médecin ophtalmologiste, avec une anesthésie locale de l'oeil, au moyen d'un collyre anesthésique. Egalement, malgré une sensibilité satisfaisante, les causes d'erreur de mesure sont nombreuses : variabilité liée aux larmes , à l'accommodation, à l'épaisseur de la cornée.

Un deuxième type de tonomètre par aplanation, encore appelé tonomètre sans contact, utilise un bref jet d'air pour déformer la cornée jusqu'à la rendre concave , mais en passant par une phase où la surface aplanie sur la cornée présente le meilleur angle de réflexion entre la source lumineuse et un capteur optoélectronique ; c'est ce maximum qui est détecté et considéré comme moment de la mesure. Le temps écoulé entre le début du jet d'air et la réflexion maximum sur la cornée peut être traduit en une valeur de pression intra-oculaire. Ce type de tonomètre impose là encore de manière préjudiciable une déformation mécanique de la cornée.

Plus récemment, des dispositifs de mesures de la pression intra-oculaire par interférométrie laser on été proposés, avec pour avantage principal qu'ils évitent la mise en oeuvre d'une force de pression mécanique exercée sur la cornée en vue de sa déformation pendant la mesure. Ces dispositifs reposent sur une mise en vibration de l'oeil au moyen par exemple d'une onde acoustique et sur l'utilisation d'un interféromètre de type Michelson pour la détection des fréquences des modes propres de vibration de l'oeil. Des travaux ont en effet montré qu'il y avait une relation simple entre les fréquence des modes propres de vibration de l'oeil et la pression intra-oculaire.

Un exemple de ce type de dispositif est décrit dans la demande de brevet internationale WO-A-9321820 Dans ce type d'interféromètre, le faisceau laser incident principal est divisé par un miroir diviseur (référencé 46 dans l'exemple de réalisation de la figure 2 de WO-A-9321820) en deux faisceaux incidents secondaires orientés à 90°, l'un des faisceaux incidents secondaires étant réfléchi par la surface de la cornée de l'oeil, et l'autre faisceau incident secondaire étant réfléchi par un miroir (référencé 70 .dans l'exemple de réalisation de la figure 2 de WO-A-9321820). Les deux faisceaux retours réfléchis respectivement par la cornée et par le miroir reviennent sur le miroir diviseur où ils interfèrent. Un inconvénient important de ce dispositif est que pour obtenir les interférences, il est impératif que les faisceaux retours soient parfaitement colinéaires. Il en résulte que la cornée doit être parfaitement alignée par rapport au miroir diviseur, et qu'il n'est pas possible de tolérer le moindre désalignement latéral ou longitudinal de la cornée, ce qui rend la mesure très contraignante en terme de positionnement de l'oeil. Egalement il en résulte que ce dispositif est extrêmement sensible aux moindres perturbations extérieures susceptibles de modifier très légèrement l'alignement du système optique, et est notamment très sensible aux moindres mouvements ou vibrations mécaniques du dispositif. Un autre inconvénient de ce type de dispositif est que la détection des fréquences propres de vibration de l'oeil par mesure de l'intensité transmise par l'interféromètre nécessite en pratique la mise en oeuvre d'un laser de forte puissance, avec le risque d'une lésion de la cornée. Enfin dans la demande de brevet internationale WO-A-9321820, l'excitation de l'oeil pour sa mise en vibration est mise en oeuvre selon une méthode harmonique, par un balayage en fréquences de l'onde acoustique d'excitation. Cette méthode d'excitation harmonique présente l'inconvénient d'augmenter la durée de la mesure, et surtout l'onde acoustique peut être traumatisante pour le tympan.

L'invention vise à proposer un nouveau procédé de détection, par interférométrie laser, d'un ou plusieurs modes propres de vibration d'un oeil, mais qui comparativement aux méthodes précitées basées sur la mise en oeuvre d'un interféromètre de type Michelson, présente principalement les avantages d'être moins contraignant pour le positionnement de l'oeil, d'être moins sensible aux perturbations extérieures et notamment aux vibrations mécaniques et de pouvoir être mis en oeuvre avec un laser de plus faible puissance.

Plus particulièrement, les avantages précités de l'invention rendent possible la réalisation d'un dispositif de détection de type ambulatoire, étant précisé que l'invention n'est toutefois pas limitée à cet aspect ambulatoire.

Le procédé de détection de l'invention comporte les étapes consistant à positionner l'oeil par rapport à un dispositif de détection en sorte de former avec ce dispositif une cavité de Pérot-Fabry, comportant deux faces réflectives opposées selon l'axe optique principal de la cavité, une de ces deux faces étant constituée par un élément réflectif fixe et l'autre étant formée par la cornée de l'oeil; injecter dans cette cavité un faisceau laser incident, les alignements latéral et longitudinal de la cornée par rapport à l'axe optique principal de la cavité étant réalisés en sorte d'obtenir des interférences longitudinales entre les faisceaux laser aller et retour réfléchis entre les deux faces réflectives de la cavité ; détecter au moyen d'un détecteur optoélectronique l'intensité (I) dans le temps de ces interférences.

De façon caractéristique, le faisceau laser incident est centré et aligné sur l'axe optique principal de la cavité Pérot-Fabry.

D'autres caractéristiques et avantages de l'invention apparaîtront plus clairement à la lecture de la description ci-après d'une variante de réalisation, laquelle est donnée à titre d'exemple non limitatif et en référence aux dessins annexés sur lesquels :

- La figure 1 est un schéma de principe d'un appareil conforme à l'invention, permettant la mesure d'au moins une fréquence propre de vibration d'un oeil et par là-même la mesure de la pression intra-oculaire (PIO),
- La figure 2 illustre un exemple (connu) de variation en fréquences des différents modes propres de vibration d'un oeil en fonction de la pression intra-oculaire,
- Les figures 3, 4, 5 et 6 illustrent un exemple de réalisation, sous forme de modules assemblés, d'un dispositif de détection selon l'invention,
- La figure 7 illustre un exemple particulier de signal mesuré par le détecteur de l'invention, et représentatif d'une fréquence propre de vibration de l'oeil de 300Hz,
- La figure 8 représente le spectre de puissance du signal de la figure 7, après transformée de Fourier,
- La figure 9 est un graphique permettant de déterminer dans un cas particulier la sensibilité longitudinale du dispositif de détection,
- La figure 10 est un schéma optique simplifié permettant d'illustrer les effets d'un désalignement latéral $\Delta_d$ de l'oeil,
- Les figures 11 et 12 représentent respectivement la hauteur h, et l'angle $\alpha$ (pour un aller-retour du faisceau lumineux dans la cavité de Pérot-Fabry) en fonction du désalignement latéral $\Delta_d$,
- Les figures 13 et 14 sont comparables respectivement aux figures 11 et 12, mais sont données pour deux aller-retour du faisceau lumineux dans la cavité de Pérot-Fabry,
- La figure 15 illustre un exemple de mire utilisée dans une première variante de réalisation pour réaliser l'alignement latéral et l'alignement longitudinal de l'oeil par rapport à l'axe optique principal du dispositif de détection,
- La figure 16 est une représentation schématique d'une autre variante de réalisation d'un appareil selon l'invention, mettant en oeuvre des moyens optiques perfectionnés pour le contrôle des alignements latéral et longitudinal de l'oeil.

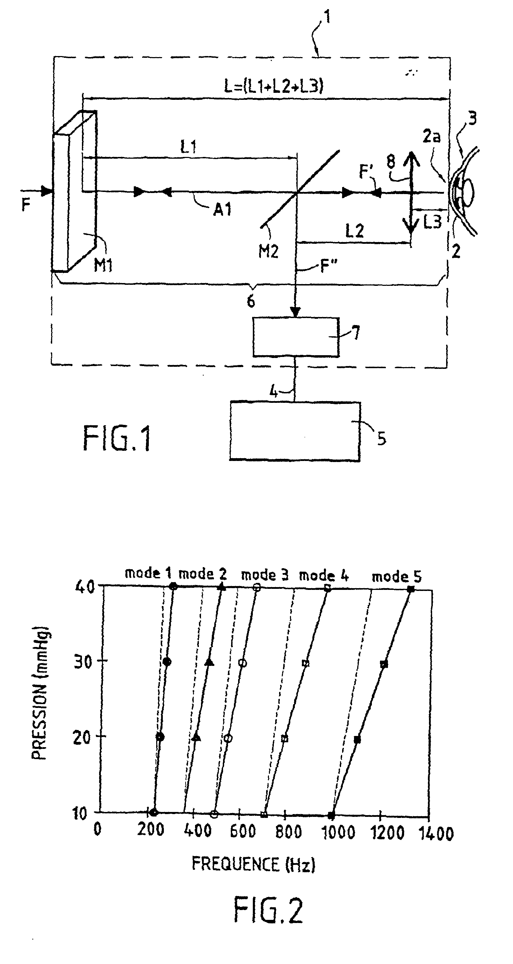

L'appareil de la figure 1 comprend :

- un dispositif 1 qui permet une détection optoélectronique de la vibration de la cornée 2 d'un oeil 3, par interférométrie laser, et qui délivre un signal électrique 4 temporel caractéristique de l'amplitude de la vibration de la cornée 2 dans le temps,
- un système électronique 5 de traitement de signal, qui est conçu pour dans une première étape calculer le spectre fréquentiel du signal temporel 4, par exemple en calculant la transformée de Fourier du signal 4, et pour dans une deuxième étape, à partir de ce spectre fréquentiel, déterminer la fréquence d'au moins un mode propre de vibration de l'oeil.

L'apport de l'invention réside essentiellement dans le dispositif de détection 1 décrit en détail ci-après. Les deux étapes de calculs précitées réalisées par le système électronique 5 et les moyens nécessaires à leur mise en oeuvre étant connus de l'homme du métier dans le domaine du traitement de signal, ils ne seront pas détaillés dans la présente description.

Préalablement à la description du dispositif de détection 1, il est rappelé que l'oeil peut être modélisé comme une hétéro-structure de forme sphérique, caractérisée par des coefficients d'élasticité, de viscosité et d'impédance. Si on excite mécaniquement l'oeil de manière appropriée pour le mettre en vibration, il est possible d'exciter les fréquences propres de vibration de cette hétéro-structure. Or ces modes propres de vibration ont été calculés par une modélisation mathématique de l'oeil, et ont permis de quantifier les variations de fréquence propres en fonction de la pression intra-oculaire. A titre d'exemple, la figure 2 annexée montre la variation en fréquences des différents modes propres de vibration en fonction de la pression intra-oculaire pour un oeil modélisé mathématiquement au moyen d'un modèle linéaire (courbes en pointillés) ou au moyen d'un modèle non linéaire (courbes en trait plein). Il est par conséquent connu à ce jour que la mesure de la fréquence de vibration d'au moins un mode propre de vibration de l'oeil permet d'accéder à la mesure de la pression intra-oculaire par l'intermédiaire d'un étalonnage à l'aide de techniques connues. De préférence, on mesure la fréquence de vibration des modes les plus élevés, et en particulier du mode (5) de la figure 2. Cette mesure de la pression intra-oculaire permet le diagnostic de pathologie ophtalmologiques, dont la principale est le glaucome de l'oeil.

Dispositif de détection optoélectronique, par interférométrie laser

En référence à la figure 1, et conformément à une caractéristique essentielle de l'invention, le dispositif de détection 1 comporte une partie optique 6, qui une fois positionnée par rapport à l'oeil forme un interféromètre de Pérot-Fabry comportant une cavité de réflexion des ondes dont l'une des deux extrémités est constituée par la surface externe 2a de la cornée 2 de l'oeil, et un détecteur optoélectronique 7 permettant de détecter l'intensité de l'onde (I) transmise en sortie par l'interféromètre 6, et de délivrer le signal électrique 4 précité fonction de cette intensité.

En référence aux figures 1 et 3 à 6, dans une variante particulière de réalisation, le dispositif de détection 1 est constitués de neuf modules A à I.

*Module A :* ce module comporte une diode laser de longueur d'onde $\lambda$ (valant par exemple 635 manomètres) équipée d'un système de stabilisation en température et en puissance (1.9 mW). La diode laser dispose d'une optique de collimatation limitant la divergence du faisceau laser. Elle supporte une alimentation de 4,5 V avec une pile ou 5 V avec une alimentation extérieure , et un courant d'alimentation limité à 60 mA.

*Module B :* ce module est formé par une lame quart d'onde (non représentée sur la figure 1) permettant de polariser circulairement le rayonnement de la diode laser.

*Module C:* il est formé par un miroir plan M1 dont les coefficients de réflexion $R_1$ et de transmission $T_1$ en intensité valent respectivement 98 % et 2 %. Son action permet :

- de limiter la perte d'énergie de la cavité Fabry-Perot due au faible coefficient de réflexion de l'oeil ($R_3$ : 2.5 %) et d'améliorer ainsi le contraste du système interférométrique.
- de limiter la puissance de la diode laser à (19.73+-0.01) $\mu$W (valeur mesurée derrière le miroir) afin de pouvoir répondre aux normes de puissance admissible sur l'oeil.

*Module D :* il est formé par un miroir diviseur M2, dont les coefficients de réflexion $R_2$ et transmission $T_2$ en intensité valent respectivement 40% et 60%. Ce miroir diviseur M2 permet de réduire une deuxième fois la puissance de la diode laser en la faisant passer à 12.8 $\mu$W (valeur mesurée après le passage de M2)

*Module E :* il est formé par une lentille de correction plan-convexe 8 de focale (f) valant plus particulièrement 30 mm. Son action permet de corriger la divergence causée par la courbure de la cornée de l'oeil. Le choix de la focale se fait en fonction de la réduction de la sensibilité au désalignement latéral de l'oeil par rapport à l'axe optique du système interférométrique. Par ailleurs, la lentille possède de préférence un traitement antireflet pour la longueur d'onde du laser, afin d'éviter les réflexions parasites au niveau de la lentille qui pourraient causer un phénomène de double cavité.

*Module F:* ce module constitue un filtre interférentiel (non représenté sur la figure 1). C'est une lame diélectrique à faces parallèles partiellement réfléchissantes qui ne transmet qu'une bande spectrale du rayonnement incident. Plus particulièrement, dans un exemple de réalisation, cette lame permet de transmettre une bande spectrale de 10 nanomètres centrée sur la longueur d'onde du laser, isolant ainsi le détecteur 7 de tout autre rayonnements optique (éclairage par exemple) que celui de la diode laser du module A. Ce filtre possède de préférence un traitement antireflet à la longueur d'onde du laser (635 nanomètres) sur sa face opposée (celle du côté du détecteur 7) afin d'éviter toutes réflexions parasites entre la surface du détecteur 7 et celle du filtre interférentiel.

*Module G :* Il forme le détecteur optoélectronique 7 précité. Plus particulièrement, ce détecteur est un détecteur à avalanche présentant les caractéristiques de fonctionnement ci-après, données pour une température de fonctionnement de 25° C et sous une alimentation +/-12V.

| PARAMETRES | VALEURS | UNITES |
|---|---|---|
| diamètre de la surface active du détecteur | 3,0 | mm |
| réponse spectrale | 400 à 1000 | nm |

(suite)

| PARAMETRES | VALEURS | UNITES |
|---|---|---|
| pic de sensibilité | 800 | nm |
| photosensivité (800 nm, gain=1) | 0,5 | A/W |
| stabilité du gain en température (25° C +/- 10° C, gain = 30) | +/- 2,5 Typ. +/- 5 Max | % |

| PARAMETRES | Min. | Typ. | Max. | Unité |
|---|---|---|---|---|
| Fréquence de coupure supérieure | 9 | 10 | - | KHz |
| Fréquence de coupure inférieure | - | DC | - | KHz |
| puissance équivalente au bruit à 800 nm (NEP) | - | 0.02 | 0.04 | $Pw/Hz^{1/2}$@10MHz |
| résistance du circuit équivalent | - | 10 | - | $M\Omega$ |
| sensibilité photoélectrique (800 nm et gain = 30 | - 1,4 | -1,5 | -1.6 | $10^6$V/W |
| intensité d'entrée maximum | 0,05 | 0,06 | - | $\mu$W |
| intensité minimum détectable | - | 0,005 | 0,01 | nW |

Il permet une intégration sur une large surface circulaire (diamètre 3.0 mm) pour pouvoir remédier au problème de désalignement optique de l'oeil. Il possède une bande passante de 100 kHz et permet de mesurer une puissance limite de $0.005.10^{-9}$ W pour la longueur d'onde de 800 nm.

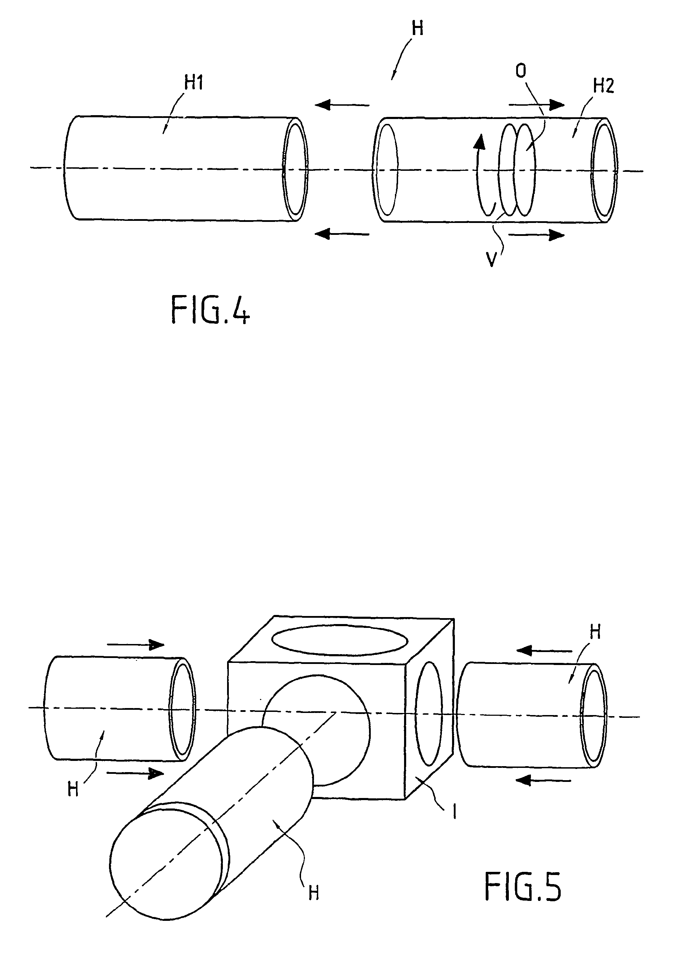

*Module H :* (figure 4) Il forme une monture tubulaire comportant un tube extérieur H1 dans lequel vient s'insérer un tube interne H2, et qui permet de supporter une optique (O) par l'intermédiaire d'une vis de serrage V. Une monture tubulaire H est conçue pour être assemblée avec d'autres monture du même type et ainsi permettre l'élaboration d'une structure plus complexe en mettant à l'abri les différentes optiques (O). Pour la réalisation du dispositif de détection 1, on utilise quatre montures tubulaires H, servant de logement respectivement à la lame quart d'onde (module B précité), au miroir plan M1 (module C), à la lentille plan-convexe 8 (module E) et au filtre interférentiel (module F)

*Module I:* (figure 5) Ce module permet d'assembler les montures tubulaires H suivant deux axes perpendiculaires et sert de logement au miroir diviseur M2.

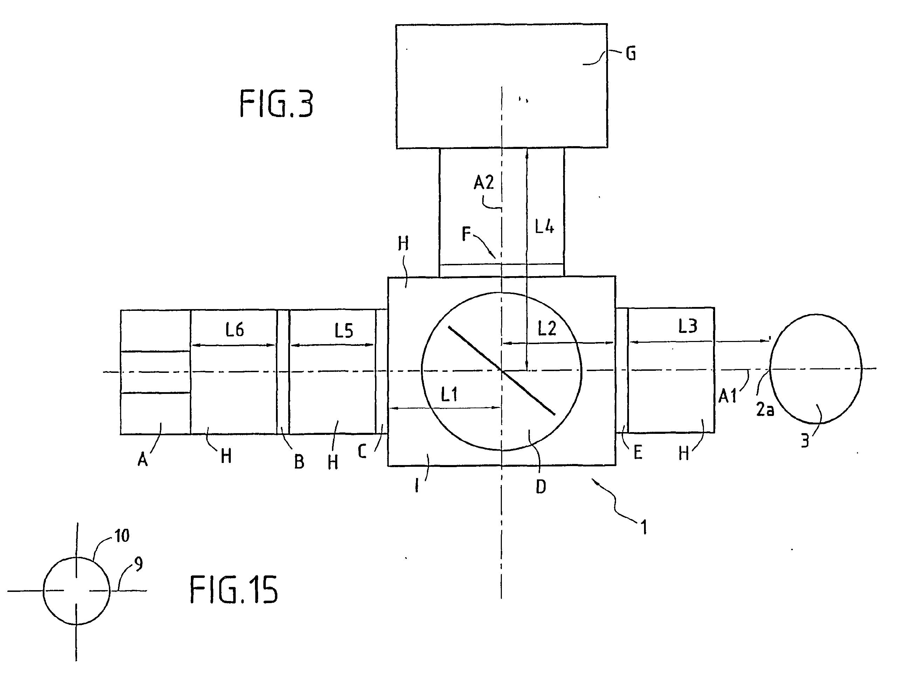

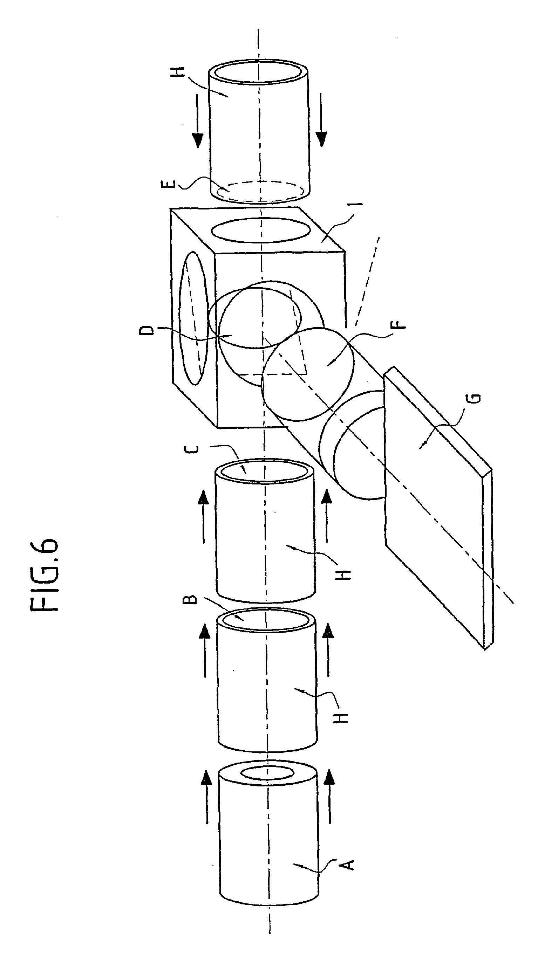

L'assemblage des différents modules entre eux pour constituer le dispositif de détection 1 est représenté sur la figure 6. En référence aux figures 1 et 6, le dispositif de détection 1 forme un système optique comportant un axe optique principal A1, et un axe optique secondaire A2 orienté à 90° du fait de la mise en oeuvre du miroir diviseur M2. La diode laser(module A), la lame quart d'onde module B), le miroir plan M1, le miroir diviseur M2 ( module C) et la lentille plan convexe 8 (module E) sont alignés de manière fixe et précise et centrés sur l'axe optique principal *A1*. Le miroir diviseur M2 ( module D), le filtre interférentiel (module F) et le détecteur 7 (module G) sont alignés de manière fixe et précise et centrés sur l'axe optique secondaire A2. Dans un exemple précis de réalisation donné à titre purement indicatif, les distances $L_1, L_2$, et $L_5$ et $L_6$ (figure 3 et figure 1) valaient 2,5cm, et la distance $L_4$ valait 5cm.

Pour détecter les modes propres de vibration d'un oeil 3 au moyen du dispositif de détection 1 qui vient d'être décrit , on aligne sensiblement ( voir discussion ci-après sur la sensibilité latérale du dispositif) le centre de la cornée 2 de l'oeil 3 sur l'axe optique principal A1, en le positionnant longitudinalement à une distance $L_3$ prédéterminée ( voir discussion ci-après sur la sensibilité longitudinale du dispositif) de telle sorte que le module I, et le module H (servant de logement à la lentille plan-convexe 8) forme une cavité de Pérot-Fabry, d'axe optique principal A1 et dont l'une des faces réflective selon cet axe optique principal est constituée par le miroir plan M1 et dont l'autre face réflective opposée est constituée par la cornée 2 de l'oeil 3, la longueur L (distance séparant la cornée du miroir plan M1) de cette cavité Pérot-Fabry étant variable, et dépendant des amplitudes de vibration de la cornée 2 de l'oeil 3.

En référence au schéma de principe de la figure 1, en fonctionnement, le faisceau laser entrant F ( après polarisation circulaire par la lame quart d'onde précitée) est injecté à l'intérieur de la cavité de Pérot-Fabry en passant à travers le miroir M1, traverse le miroir diviseur M2 jusqu'à atteindre la surface 2a de la cornée 2. Une partie de ce faisceau incident est réfléchie par la cornée jusqu'au miroir diviseur M2 sous la forme d'un faisceau retour F'. Ce faisceau retour F' lorsqu'il atteint le miroir diviseur M2 est en partie dévié à 90° par le miroir diviseur M2, pour former le faisceau sortant F"; l'autre partie du faisceau retour F' étant transmise à travers M2 jusqu'au miroir plan M1, où elle subit une réflexion etc....

L'intensité transmise par cette cavité Pérot-Fabry de longueur L variable , c'est-à-dire l'intensité I du faisceau sortant F" par rapport à l'intensité $I_0$ (connue) du faisceau laser incident F, est donnée par la formule suivante :

$$(1)\quad \frac{I}{I_0} = \frac{T_1 R_2}{T_2 R_1 (1 - T_2\sqrt{R_1 R_3})^2} * \frac{1}{1 + \dfrac{4T\sqrt{R_1 R_3}}{(1 - T_2\sqrt{R_1 R_3})^2}\sin^2 \dfrac{2\pi}{\lambda}L}$$

L'intensité (I) en sortie de la cavité Pérot-Fabry ( signal 4 délivrée par le détecteur optoélectronique 7) varie de manière périodique en fonction de la longueur L variable de la cavité.

Pour des multiples entiers de λ/2 (λ longueur d'onde du laser ), l'intensité est maximale et donnée par la formule ci-après :

$$(2)\quad I_M = I_0 * \frac{R_2 T_1}{R_1 T_2 (1 - T_2\sqrt{R_1 R_3})^2}$$

Pour des multiples de entiers et un demi de λ/2, l'intensité est minimale et donnée par la formule ci-après :

$$(3)\quad I_m = I_0 * \frac{R_2 T_1}{R_1 T_2 (1 - T_2\sqrt{R_1 R_3})^2} * \frac{1}{1 + \dfrac{4T_2\sqrt{R_1 R_3}}{(1 - T_2\sqrt{R_1 R_3})^2}}$$

Ainsi, la période de la fonction de transfert de l'interféromètre Pérot-Fabry correspond à la moitié de la longueur d'onde du faisceau laser injecté dans la cavité. Autrement dit, pour une diode laser de longueur d'onde λ, l'interféromètre permet de mesurer des variations de longueur L de l'ordre de λ/2.

On peut assimiler les vibrations de la cornée 2 à une oscillation sinusoïdale amortie (pour tenir compte de la contrainte exercée par l'orbite de l'oeil et de celle des muscles associés à l'oeil). Dans ce cas, la longueur L de la cavité prend la forme :

$$(4)\quad L(t) = L_0 + l.e^{\frac{-t}{c}}.\sin(wt)$$

Dans cette formule (4) :

- l représente l'amplitude de vibration de la surface 2$\underline{a}$ de la cornée 2
- w est la fréquence de vibration de l'oeil,
- c est le coefficient d'amortissement.

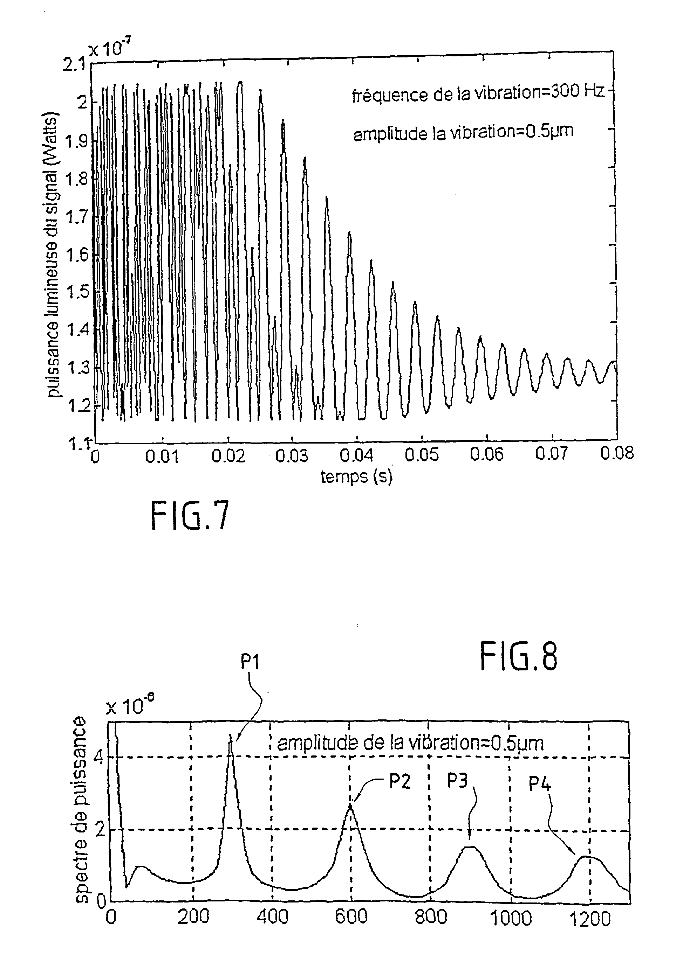

Des considérations ci-dessus, il résulte que l'intensité (I) en sortie de la cavité Pérot-Fabry et mesurée par le détecteur optoélectronique 7 (signal 4) passe par une succession d'extremums à une certaine vitesse caractéristique de la fréquence de vibration de la cornée. A titre indicatif, un exemple particulier de signal 4 mesuré par le détecteur est illustrée sur la figure 7, et représentatif d'une fréquence propre de vibration de l'oeil de 300Hz et avec une amplitude initiale d'oscillation de la cornée avant amortissement de 0,5μm

Pour accéder à la fréquence de vibration de la cornée, il suffit d'effectuer la transformée de Fourier du signal 4 (première étape réalisée par le système électronique 5 de la figure 1.) On obtient ainsi un spectre de puissance du signal 4, du type de celui illustré sur la figure 8. On retrouve dans ce spectre, un pic principal ($P_1$) correspondant à la

fréquence de vibration de la cornée 2, ainsi que les harmoniques de cette fréquence, caractérisées par des pics ($P_2$, $P_3$, $P_4$) de plus faible intensité.

L'oeil étant un élément non fixe de la cavité Pérot-Fabry, son alignement constitue un paramètre variable d'une mesure à l'autre, pour lequel il convient de définir les limites de variations à ne pas dépasser pour obtenir la mesure des modes propres de vibration de l'oeil. La sensibilité du dispositif de détection 1 précédemment décrit à un désalignement longitudinal et à un désalignement latéral vont à présent être succinctement abordés.

Sensibilité longitudinale

L'objectif est de déterminer une plage de stabilité qui permet de garantir une refocalisation du faisceau selon l'axe optique principal A1 sur lui même dans la cavité. Pour la stabilité longitudinale, il faut considérer la partie composée du miroir plan M1 et du système oeil - lentille 8. Sachant que dans le dispositif de détection 1 seule la distance oeil -lentille 8 est variable, l'étude de la stabilité longitudinale consiste à déterminer la capacité de la cavité Pérot-Fabry ( formée par le miroir plan M1 et le système oeil / lentille 8) à confiner le rayonnement en fonction de la distance oeil / lentille 8 (distance $L_3$ sur la figure 1).

La cavité Pérot-Fabry est stable longitudinalement lorsqu'après n aller-retour, le faisceau est refocalisé sur lui même. En modélisant l'oeil par un miroir sphérique divergent de rayon de courbure R, la matrice de transfert de cette cavité de Pérot-Fabry, permettant de calculer les coordonnées du rayon lumineux après n aller-retour dans la cavité en fonction des coordonnées initiales à l'entrée de la cavité est :

$$(5) \quad M = \frac{1}{\sin\theta} \begin{bmatrix} A\sin n\theta - \sin(n-1)\theta & B\sin\theta \\ C\sin\theta & D\sin n\theta - \sin(n-1)\theta \end{bmatrix}$$

avec d'une part :

$$(6) \quad A = \left(1-\frac{L_3}{f}\right) * \left[\left(1-\frac{L}{f}\right)\left(1+\frac{2L_3}{R}\right)+\frac{2L}{R}\right] - \frac{1}{f}\left[L_3\left(1-\frac{L}{f}\right)+L\right]$$

$$(7) \quad B = \left[\left(1-\frac{L}{f}\right)\left(1+\frac{2L_3}{R}\right)+\frac{2L}{R}\right]\left[L\left(1-\frac{L_3}{f}\right)+L_3\right] + \left(1-\frac{L}{f}\right)\left[L_3-\left(1-\frac{L}{f}\right)+L\right]$$

$$(8) \quad C = \left[-\frac{1}{f}\left(1+\frac{2L_3}{R}\right)+\frac{2}{R}\right]\left(1-\frac{L_3}{f}\right)-\frac{1}{f}\left(1-\frac{L_3}{f}\right)$$

$$(9) \quad D = \left[L\left(1-\frac{L_3}{f}\right)+L_3\right]\left[\frac{2}{R}-\frac{1}{f}\left(1+\frac{2L_3}{R}\right)\right]+\left(1-\frac{L_3}{f}\right)\left(1-\frac{L}{f}\right)$$

Et avec d'autre part :

$$(10) \quad \cos\theta = \frac{1}{2}(A + D)$$

Il est rappelé que les distances L et $L_3$ ci-dessus sont celles indiquées sur la figure 1 et que le paramètre (f) représente la distance focale de la lentille 8.

Pour que la cavité soit stable, il faut que θ soit réel, cette condition de stabilité étant équivalente à la condition : $|A + D| \leq 2$.

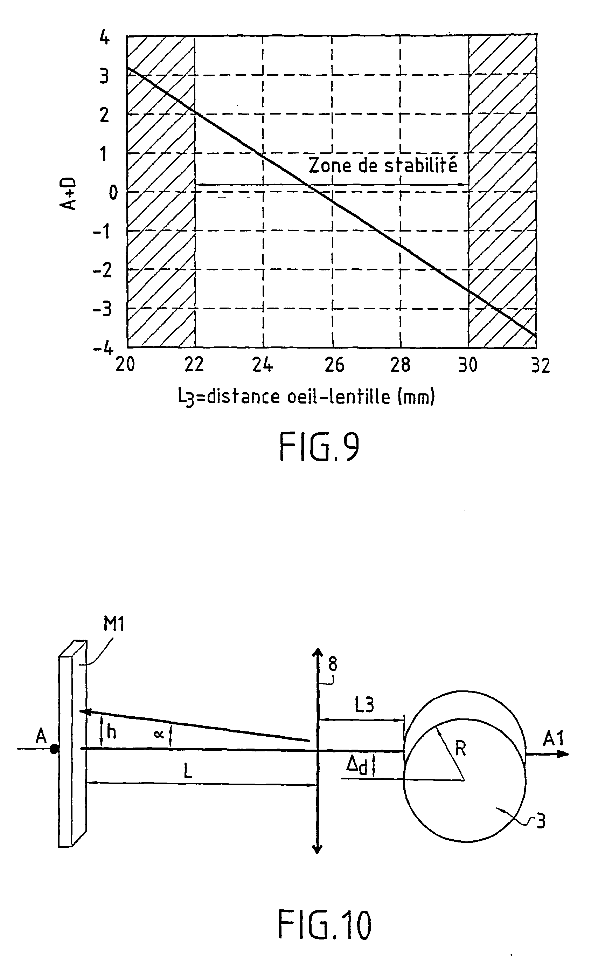

On a représenté, sur la figure 9, la courbe A +D en fonction de la distance oeil / lentille 8 ($L_3$), pour une focale f de la lentille 8 valant 30mm. On peut constater sur ce graphique que dans ce cas particulier la zone de stabilité est obtenue pour $L_3$ comprise entre 22mm et 30mm. Pour un bon fonctionnement du dispositif de détection 1 , il suffit dans ce cas de s'assurer que l'oeil est maintenu à distance $L_3$ comprise dans la plage de stabilité précitée.

Sensibilité latérale

L'alignement latéral de l'oeil avec l'axe optique principal A1 de la cavité Pérot-Fabry est primordial pour l'obtention d'un signal d'interférence sur le détecteur 7. L'objectif de l'étude ci-après de la sensibilité latérale est d'une part de déterminer un seuil limite de tolérance au désalignement latéral et d'autre part de concevoir un système d'alignement de l'oeil tenant compte de cette sensibilité latérale.

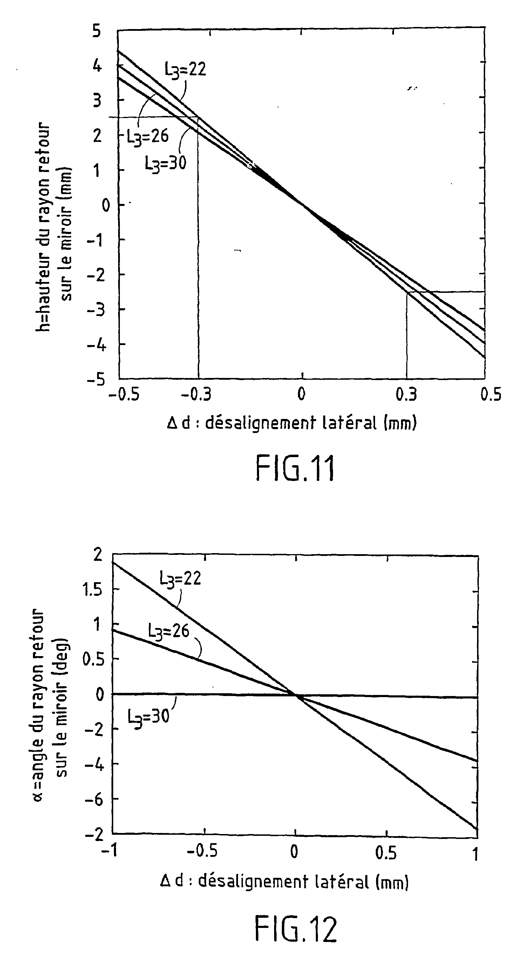

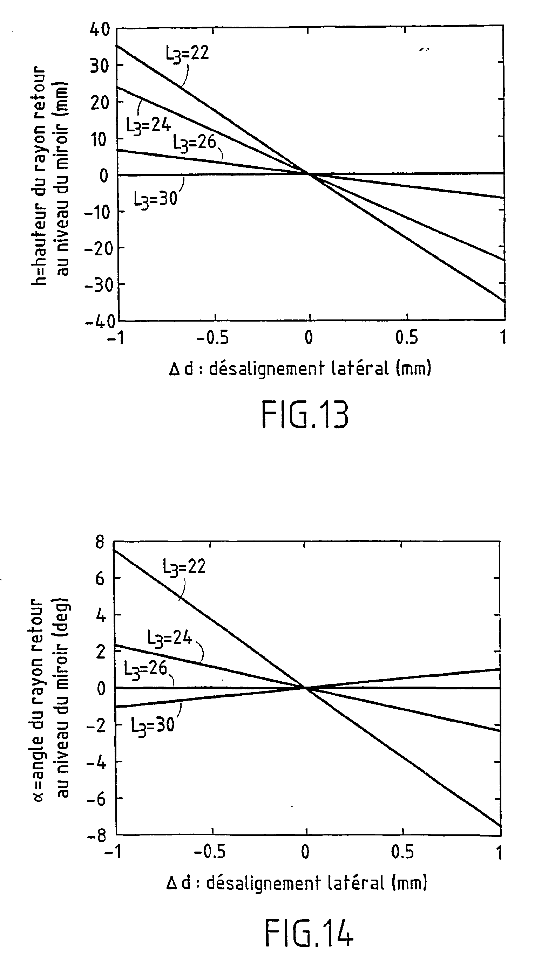

En référence au schéma optique de la figure 10, le paramètre caractérisant ce désalignement latérale est la distance $\Delta_d$, laquelle a une influence sur la hauteur h et l'angle α du rayon retour sur le miroir plan M1. On a représenté sur les figures 11 et 12 respectivement la hauteur h et l'angle α après un aller-retour du faisceau lumineux dans la cavité en fonction du désalignement latéral $\Delta_d$ par rapport à l'axe optique A1 pour une focale f valant 30mm, et ce pour plusieurs distance oeil - lentille 8 (distance $L_3$) en mm. Les figures 13 et 14 sont comparables respectivement aux figures 11 et 12 mais pour deux aller-retour du faisceau lumineux dans la cavité. En pratique, il n'est pas nécessaire de considérer plus de deux aller-retour du faisceau, car au delà les amplitudes des ondes qui interfèrent deviennent négligeables.

Les résultats des figures 11 à 14 montrent que le dispositif de détection 1 est très sensible au désalignement latéral. Un désalignement latéral de 0,5mm de l'oeil par rapport à l'axe optique fait diverger le faisceau retour d'un angle allant de 2° à 0° selon que l'oeil est placé à une distance $L_3$ de la lentille 8 comprise entre 22mm et 30mm. Cette divergence se traduit par une hauteur du faisceau h au niveau du miroir plan M1 pouvant atteindre 4mm. On peut considérer que la cavité est stable si la hauteur h est inférieure à la largeur (plus communément appelée " waist ") du faisceau dans la cavité. En conséquence, dans le cas particulier des figures 11 à 14 et avec un faisceau de largeur moyenne 3mm, on peut considérer que le dispositif est stable jusqu'à un désalignement latéral $\Delta_d$ de 0,3mm, et qu'au delà la cavité Pérot-Fabry devient instable.

Optimisation dry dispositif de détection pour réduire la sensibilité au désalignement latéral

La réduction de la sensibilité latérale peut être obtenue premièrement par la réduction de la longueur L de la cavité Pérot-Fabry. Une cavité plus courte permet de limiter les effets de divergence du faisceau retour (paramètres h et α) induits par le désalignement latéral de l'oeil.

Un réduction de sensibilité latérale plus conséquente peut également être obtenue par le choix d'une lentille 8 possédant un courte focale (f). Les calculs effectués pour une focale de 20mm (au lieu de 30mm précédemment) montrent une diminution de hauteur h de plus de 1 mm pour un aller-retour et de plus de 5mm pour deux aller-retour. Ainsi le choix d'une focale de 20mm pour la lentille 8 permet d'aligner l'oeil par rapport à l'axe optique avec un précision moins contraignante de 0,5mm. A l'inverse, pour illustrer l'importance de la focale de la lentille 8 sur le la sensibilité latérale, si on équipe le dispositif de détection 1 avec une lentille 8 de focale valant 50mm, l'alignement l'oeil avec l'axe optique devra être réalisé avec une précision inférieure à 0,15mm.

En conclusion, en choisissant judicieusement une focale courte pour la lentille 8 (par exemple 20mm) et une cavité de longueur L courte, on optimise la sensibilité latérale du dispositif.

Perfectionnement du dispositif de détection 1 : mire permettant les alignements latéral et longitudinal

On a représenté sur la figure 15, le motif d'une mire à l'échelle 1 qui comprend une croix 9 utilisée pour l'alignement latéral de l'oeil avec l'axe optique et un cercle 10 utilisé pour l'alignement longitudinal de l'oeil par rapport à la lentille 8 (distance $L_3$). Cette mire est placée dans la cavité Pérot-Fabry, entre l'élément réflectif M1 et la lentille (8), et plus particulièrement entre le miroir diviseur M2 et la lentille 8, de préférence au niveau du plan focal de la lentille 8, afin que l'observation par l'oeil de l'image de la mire à travers la lentille 8 soit la moins fatigante possible, un oeil normal n'ayant pas besoin d'accommoder. La mire se trouvant sur le trajet du faisceau, elle est réalisée sur une lame transparente possédant un coefficient de réflexion, à la longueur d'onde λ du laser, le plus faible possible, afin de ne pas diminuer le niveau de l'intensité transmise par la cavité. Dans ce but, on traitera par exemple la lame avec un antireflet à la longue d'onde du laser.

<u>Alignement latéral</u>

Lorsque l'oeil observe l'image virtuelle de la croix 9 de la mire à travers la lentille 8, les branches de la croix se déforment lorsque l'oeil n'est plus aligné avec l'axe optique de lentille 8 ( c'est-à-dire l'axe optique A1 de la cavité). Cette déformation est d'autant plus importante que la focale (f) de la lentille est courte.

<u>Alignement longitudinal</u>

La distance minimale $L_3$ (valeur inférieure de la plage de stabilité longitudinale précédemment définie) est fixée par la longueur de la monture tubulaire dans la quelle est insérée la lentille 8. Il suffit donc pour l'alignement longitudinal de pouvoir majorer la distance $L_3$. (distance oeil - lentille 8) à la valeur maximale autorisée (30mm dans l'exemple précité) ) de la plage de stabilité longitudinale du dispositif. A cet effet, le diamètre du cercle 10 de la mire est déterminée en fonction de cette valeur maximale autorisée de $L_3$, en sorte qu'il soit observable par l'oeil 3 à travers la lentille 8, lorsque l'oeil est située à une distance $L_3$ de la lentille 8 inférieure à la valeur maximale de la plage de stabilité. A titre d'exemple, pour une valeur maximale de 30mm, le diamètre du cercle 10 sera de 1 cm.

<u>Mise en</u> oeu<u>vre du dispositif de détection</u>

Au moyen de la mire, on commence par aligner l'oeil latéralement par rapport l'axe optique A1 de la cavité Pérot-Fabry (correction de l'alignement latéral jusqu'à ce que croix 9 de la mire n'apparaisse plus déformée) et longitudinalement par rapport à la lentille ( correction de la distance $L_3$ jusqu'à ce que l'oeil puisse observer le cercle 10 de la mire. Pour les corrections en position de l'oeil, le dispositif pourra être fixé sur un appareil permettant un réglage précis en position du dispositif 1 dans un espace tridimensionnel et comportant un système de contention de la tête du patient dans cet espace pour éviter tout déplacement parasite autre que celui du dispositif de détection (Par exemple mentonnière avec palonnier pour le déplacement du dispositif de détection 1). Ceci n'est toutefois pas limitatif de l'invention ;il est en effet également envisageable de concevoir un dispositif de détection qui soit de type ambulatoire, sachant notamment que la mire permet aisément à une personne non initiée de valider les alignements latéral et longitudinal de son oeil par rapport à l'axe optique A1 du dispositif.

Une fois l'oeil aligné longitudinalement et latéralement , on réalise une excitation mécanique de l'oeil pour sa mise en vibration. Cette excitation peut être de type harmonique ou de préférence de type impulsionnelle, sachant que le dispositif de détection peut être utilisé avec l'une ou l'autre de ces deux méthodes d'excitation. Dans le premier cas (méthode harmonique) on excite mécaniquement l'oeil, par exemple au moyen d'une onde sonore transmise jusqu'à la cavité oculaire, et en effectuant un balayage en fréquences. Cette méthode harmonique présente toutefois l'inconvénient d'être longue, et traumatisante pour le patient. On préfère en conséquence, l'utilisation de la méthode impulsionnelle, qui consiste à soumettre l'oeil à une impulsion brève assimilable à un dirac couvrant tout le spectre de fréquences d'excitation. Cette excitation de type impulsionnelle peut par exemple être obtenue par un impact bref et léger sur le crâne du patient à proximité du logement de l'oeil. De manière encore plus avantageuse et inattendues selon l'invention, il a été observé et validé qu'elle pouvait être obtenue par un simple cillement de paupière, ou éventuellement plusieurs cillements brefs et répétés de la paupière. Ceci peut s'expliquer par le fait, que lors d'un cillement, la paupière frotte sur la face antérieure de l'oeil (cornée, conjonctive bulbaire et indirectement la sclère). Ce frottement bref peut ainsi être assimilé à une excitation mécanique impulsionnelle de l'oeil.

L'invention n'est pas limitée au dispositif particulier de détection qui a été décrit en référence aux figures annexées. D'une manière générale, la lentille de correction plan-convexe 8 pourrait être remplacée par toute lentille convergente. Dans une autre variante de réalisation, le miroir (M1) pourrait être remplacé par une lentille plan-convexe dont la face plane serait traitée de manière à permettre la réflexion du faisceau laser dans la cavité Pérot-Fabry. Dans une autre variante de réalisation, la cavité Pérot-Fabry pourrait être réalisée au moyen d'une fibre optique ; plus particulièrement, cette fibre optique serait équipée sur l'une des ses extrémité d'une micro-lentille, remplissant la même fonction que la lentille de correction 8 du dispositif de détection précédemment décrit, la fibre optique comporterait un réseau de Bragg par exemple, remplissant la même fonction que le miroir M1. Cette variante avec fibre optique permet avantageusement de concevoir un dispositif de détection miniaturisé, de type ambulatoire.

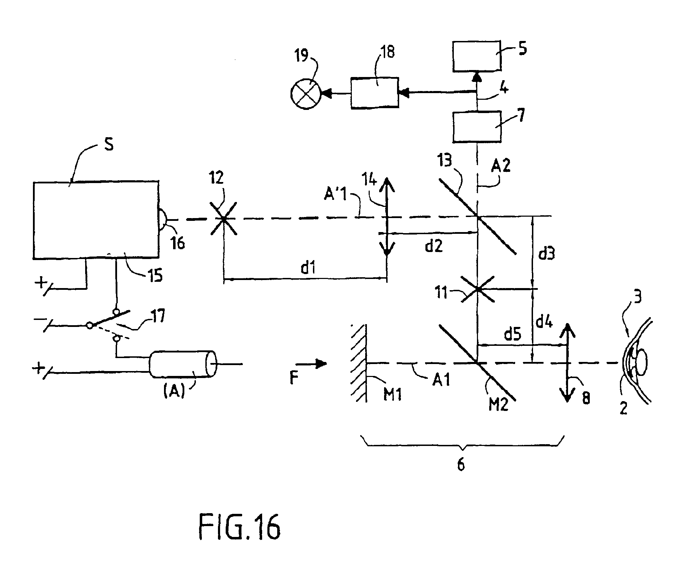

On a représenté sur la figure 16, une variante de réalisation comportant des moyens perfectionnées pour le contrôle des alignements latéral et longitudinal de l'oeil par rapport à l'axe optique principal (A1). Ces moyens perfectionnés comportent :

- une source lumineuse S qui, une fois mise sous tension, émet un faisceau incohérent, qui n'est pas dangereux pour l'oeil ; il s'agit par exemple d'une source de lumière blanche 15 équipée en sortie d'un verre dépoli 16 ;
- une lame semi-réfléchissante 13 qui est alignée avec le miroir diviseur M2, selon l'axe optique secondaire A2, et avec la source lumineuse S selon un axe optique A'1 parallèle à l'axe principal A1, et qui permet d'une part de diriger le faisceau lumineux en provenance de la source S en direction du miroir diviseur M2, ledit faisceau étant ensuite dirigé vers l'oeil selon l'axe optique principal A1, après avoir traversé la lentille 8, et qui d'autre part laisse passer à destination du détecteur 7 (axe optique secondaire A2) le faisceau retour réfléchi par l'oeil;
- une lentille 14 convergente ( par exemple plan-convexe), de focale f', centrée sur l'axe optique A'1 et positionnée entre la source S et la lentille semi-réflechissante 13,
- deux mires 11 et 12 ( ou réticules) non orientées, par exemple en forme de croix, disposée de part et d'autre de lentille 14 et centrées sur l'axe optique de la lentille 14 la mire 11 étant plus particulièrement centrée sur l'axe optique secondaire (A2) entre la lentille semi-réflechissante 13 et le miroir diviseur M2, et la mire 12 étant centrée sur l'axe optique A'1 entre la source S et la lentille 14.

De préférence, les deux mires 11 et 12 sont identiques et sont positionnées à une distance (2f') de la lentille 14 (distance $d_1$ pour la mire 12, et chemin optique ($d_2 + d_3$) pour la mire 11). De préférence, la mire 11 est positionnée à une distance (2f) de la lentille de correction 8 [figure 16 / chemin optique ($d_4 + d_5$) ]. Dans la variante préférée de réalisation de la figure 16, la distance focale (f) de la lentille de correction 8 est égale à la distance focale f' de la lentille 14. Cette caractéristique n'est toutefois pas indispensable.

Plus particulièrement, la mise sous tension de la source lumineuse S d'une part et de la diode laser (module A) d'autre part est commandée par un contacteur 17, à deux positions : une première position (illustrée en trait plein) dans laquelle seule la source S est alimentée ; une seconde position ( illustrée en pointillés) dans laquelle seule la diode laser est alimentée.

Les réglages de l'alignement longitudinal et transversal de l'oeil sont effectués au moyen exclusivement de la source lumineuse S, la diode laser n'étant pas en fonction lors de ces réglages.

Réglage de l'alignement latéral de l'oeil par rapport à l'axe optique (A1) :

La source lumineuse S étant alimentée, elle éclaire les deux mires 11 et 12, et l'alignement latéral de l'oeil lest obtenu lorsque l'oeil 2 voit les deux mires 11 et 12 confondues.

Réglage de l'alignement longitudinal de l'oeil selon l'axe optique (A1) :

L'alignement longitudinal optimal de l'oeil est obtenu, lorsque l'intensité lumineuse détectée par le détecteur 7 est supérieure à un seuil prédéterminé, et de préférence lorsqu'elle atteint son niveau maximum. Dans ce but, en référence à la figure 16, un circuit comparateur 18 effectue un comparaison entre le signal 4 délivré par le détecteur 7 et un seuil prédéterminé, de préférence réglable, et commande le déclenchement d'un indicateur 19 validant pour l'utilisateur un alignement longitudinal correct (par exemple allumage d'une diode électroluminescente) lorsque l'amplitude du signal 4 est supérieure au seuil.

Une fois les alignements latéral et longitudinal de son oeil réalisés, l'utilisateur peut au moyen du contacteur 17 mettre en service la diode laser pour la mesure de PIO. Cette variante de la figure 16 permet avantageusement de simplifier le contrôle du réglage de l'alignement latéral et longitudinal de l'oeil, préalablement à la mesure, et rend ainsi ce contrôle à la portée de l'utilisateur, sans nécessiter de faire appel à un tiers spécialisé, tel que notamment un praticien ophtalmologiste.

Dans une variante de réalisation préférée, la source lumineuse S est une source de lumière blanche. Cette caractéristique n'est toutefois pas limitative. Il pourrait s'agir d'une source lumineuse pressentant un spectre en fréquence réduit centré sur une longueur d'onde donnée, et non traumatisante pour l'oeil (par exemple une source rouge). Dans ce cas, lorsque le détecteur optoélectronique 7est équipé en amont d'un filtre interférentiel (non représenté sur la figure 1 ou 16), il convient de s'assurer que ce filtre interférentiel permet la transmission du rayonnement issue de la source S.

**Revendications**

1. Procédé de détection d'au moins un mode propre de vibration d'un oeil par interférométrie laser, ledit procédé comportant les étapes consistant à :

    - positionner l'oeil (3) par rapport à un dispositif de détection (1) en sorte de former avec ce dispositif une cavité de Pérot-Fabry d'axe optique principal (A1) et comportant deux faces réflectives opposées selon l'axe optique principal (A1), une de ces deux faces étant constituée par un élément réflectif (M1) fixe et l'autre étant formée par la cornée (2) de l'oeil (3) ;

- injecter dans cette cavité un faisceau laser (F) incident, les alignements latéral et longitudinal de la cornée (2) par rapport à l'axe optique principal (A1) étant réalisés en sorte d'obtenir des interférences longitudinales entre les faisceaux laser aller et retour réfléchis entre les deux faces réflectives de la cavité (M1,2); et

- détecter au moyen d'un détecteur optoélectronique (7) l'intensité (I) dans le temps de ces interférences, ledit procédé étant **caractérisé en ce que** le faisceau laser (F) incident est aligné et centré sur l'axe optique principal (A1) de la cavité Pérot-Fabry.

2.  Procédé selon la revendication 1, **caractérisé en ce qu'**on interpose entre l'oeil (3) et l'élément réflectif (M1) une lentille de correction (8) convergente centrée sur l'axe optique principal (A1), et permettant de réduire la sensibilité au désalignement latéral de l'oeil par rapport à l'axe optique principal (A1).

3.  Procédé selon la revendication 2, **caractérisé en ce que** la lentille de correction (8) possède une distance focale (f) courte inférieure à 50mm, et de préférence inférieure à 30mm.

4.  Procédé selon l'une des revendications 1 à 4 , **caractérisé en ce qu'**on utilise une mire (9) positionnée dans la cavité Pérot-Fabry et centrée sur l'axe optique principal (A1), et **en ce qu'**on aligne latéralement l'oeil par rapport à l'axe optique principal (A1) en sorte que la mire vue par l'oeil ne soit pas déformée.

5.  Procédé selon les revendications 2 et 4, **caractérisé en ce que** la mire (9) est positionnée entre l'élément réflectif (M1) et la lentille de correction (8), dans le plan focal de la lentille de correction (8).

6.  Procédé selon l'une des revendications 1 à 4, **caractérisé en ce qu'**on utilise une mire (10) positionnée dans la cavité Pérot-Fabry et centrée sur l'axe optique principal (A1), et **en ce qu'**on aligne longitudinalement l'oeil selon l'axe optique principal (A1) en sorte que cette mire (10) soit visible par l'oeil.

7.  Procédé selon les revendications 2 et 6, **caractérisé en ce que** la mire est (10) est positionnée entre l'élément réflectif (M1) et la lentille de correction (8), dans le plan focal de la lentille de correction (8).

8.  Procédé selon l'une des revendications 1 à 4 **caractérisé en ce qu'**on utilise une lentille convergente (14) et deux mires (11,12) disposées de part et d'autre de la deuxième lentille (14) et centrées sur l'axe optique de la lentille (14), et **en ce que** pour contrôler l'alignement latéral de l'oeil par rapport à l'axe optique principal (A1), on éclaire les deux mires (11,12) au moyen d'un faisceau lumineux incohérent, et on aligne latéralement l'oeil par rapport à l'axe optique principal (A1) en sorte que les images des deux mires vues par l'oeil (3) soient superposées.

9.  Procédé selon l'une des revendications 1 à 4 **caractérisé en ce qu'**on utilise une lentille convergente (14) et deux mires (11,12) disposées de part et d'autre de la deuxième lentille (14) et centrées sur l'axe optique de la lentille (14), et **en ce que** pour contrôler l'alignement longitudinal de l'oeil selon l'axe optique principal (A1), on éclaire les deux mires (11,12) au moyen d'un faisceau lumineux incohérent, et on contrôle l'intensité du faisceau retour réfléchi par l'oeil.

10. Procédé selon la revendication 8 ou 9 **caractérisé en ce que** les deux mires (11,12) sont identiques et positionnées à une distance (2f) de la lentille (14), f étant la distance focale de la lentille (14).

11. Procédé selon la revendication 2 et selon l'un des revendications 8 à 10, **caractérisé en ce que** l'une (11) des deux mires est positionnée à une distance (2f) de la lentille de correction (8), f étant la distance focale de cette lentille.

12. Procédé selon l'une quelconque des revendications 1 à 11, **caractérisé en ce qu'**une fois l'oeil aligné longitudinalement et latéralement par rapport à l'axe optique principal (A1), on met en vibration la cornée par un cillement ou plusieurs cillements répétés de la paupière.

13. Dispositif de détection par interférométrie laser d'au moins un mode propre de vibration d'un oeil, ledit dispositif comportant :

- une cavité de Pérot-Fabry d'axe optique principal (A1), dont l'une des deux faces réflectives est formée par un élément réflectif (M1) aligné sur l'axe optique (A1), et dont l'autre face réflective est formée, lors de l'utilisation du dispositif, par la cornée (2) de l'oeil (3) dont on souhaite détecter au moins un mode propre de vibration ;

- une source laser permettant l'émission d'un faisceau laser (F) incident sur la cavité de Pérot-Fabry; et
- un détecteur optoélectronique (7) pour la détection de l'intensité (I) des interférences longitudinales entre les faisceaux aller et retour réfléchis selon l'axe optique principal (A1) entre les deux faces réflectives (M1,2) de la cavité de Pérot-Fabry,

ledit dispositif étant **caractérisé en ce que** la source laser est positionnée de sorte que le faisceau laser (F) incident est aligné et centré sur l'axe optique principal (A1) de la cavité.

14. Dispositif de détection selon la revendication 13, **caractérisé en ce qu'**il comprend une lentille de correction (8) convergente, positionnée entre les deux faces réflectives de la cavité Pérot-Fabry, centrée sur l'axe optique principal (A1), et permettant de réduire la sensibilité au désalignement latéral de l'oeil.

15. Dispositif selon la revendication 14, **caractérisé en ce que** la lentille de correction (8) possède une focale (f) courte inférieure à 50mm, et de préférence inférieure à 30mm.

16. Dispositif selon la revendication 13 **caractérisé en ce que** pour le contrôle de l'alignement longitudinal de l'oeil selon l'axe optique principal (A1) et/ou de l'alignement latéral de l'oeil par rapport à l'axe optique principal (A1), on utilise une mire (9,10) positionnée sur l'axe optique principale (A1) entre les deux faces réflectives de la cavité Pérot-Fabry.

17. Dispositif selon les revendications 14 et 16 **caractérisé en ce que** la mire (9,10) est positionnée entre l'élément réflectif (M1) et la lentille de correction (8), dans le plan focal de la lentille de correction (8).

18. Dispositif selon la revendication 13, **caractérisé en ce que**, pour le contrôle de l'alignement longitudinal de l'oeil selon l'axe optique principal (A1) et/ou de l'alignement latéral de l'oeil par rapport à l'axe optique principal (A1), il comprend une lentille convergente (14) et deux mires (11,12) disposées de part et d'autre de la deuxième lentille (14) et centrées sur l'axe optique de la lentille (14), et une source lumineuse permettant d'éclairer les deux mires (11,12) au moyen d'un faisceau lumineux incohérent.

19. Dispositif selon la revendication 18 **caractérisé en ce que** les deux mires (11,12) sont identiques et positionnées à une distance (2f) de la lentille (14), f' étant la distance focale de la lentille (14).

20. Dispositif selon les revendications 14 et 18, **caractérisé en ce que** l'une (11) des deux mires est positionnée à une distance (2f) de la lentille de correction (8), f étant la distance focale de cette lentille.

21. Procédé de mesure de la pression intra-oculaire d'un oeil par mise en vibration et détection d'au moins un mode de vibration de l'oeil conformément au procédé visé à l'une quelconque des revendications 1 à 12.

**Claims**

1. A method of detecting at least one natural mode of vibration of an eye by laser interferometry, said method comprising the steps of:

- positioning the eye (3) relative to a detection device (1) so as to co-operate with the device to form a Fabry-Perot cavity having a main optical axis (A1) and comprising two opposite reflecting faces on the main optical axis (A1), one of these two faces being constituted by a stationary reflecting element (M1) and the other being formed by the cornea (2) of the eye (3);
- injecting an incident laser beam (F) into said cavity, the cornea (2) being aligned laterally and longitudinally relative to the main optical axis (A1) in such a manner as to obtain longitudinal interference between the go and the return laser beams reflected between the two reflecting faces of the cavity (M1,2); and
- using an optoelectronic detector (7) to detect the intensity (I) of said interference as a function of time, said method being **characterized in that** the incident laser beam (F) is aligned and centered on the main optical axis (A1) of the Fabry-Perot cavity.

2. A method according to claim 1, **characterized in that** a converging correction lens (8) is interposed between the eye (3) and the reflecting element (M1), the lens being centered on the main optical axis (A1) and serving to reduce sensitivity to lateral misalignment of the eye relative to the main optical axis (A1).

3. A method according to claim 2, **characterized in that** the correction lens (8) possesses a short focal length (f) of less than 50 mm, and preferably of less than 30 mm.

4. A method according to one of claims 1 to 4, **characterized in that** a reticle (9) positioned in the Fabry-Perot cavity and centered on the main optical axis (A1) is used, and **in that** the eye is aligned laterally relative to the main optical axis (A1) in such a manner that the reticle as seen by the eye is not deformed.

5. A method according to claims 2 and 4, **characterized in that** the reticle (9) is positioned between the reflecting element (M1) and the correction lens (8) in the focal plane of the correction lens (8).

6. A method according to one of claims 1 to 4, **characterized in that** a reticle (10) positioned in the Fabry-Perot cavity and centered on the main optical axis (A1) is used, and **in that** the eye is aligned longitudinally on the main optical axis (A1) in such a manner that this reticle (10) is visible to the eye.

7. A method according to claims 2 and 6, **characterized in that** the reticle (10) is positioned between the reflecting element (M1) and the correction lens (8) in the focal plane of the correction lens (8).

8. A method according to one of claims 1 to 4, **characterized in that** a converging lens (14) and two reticles (11,12) disposed on either side of the second lens (14) and centered on the optical axis of said lens (14) are used, and **in that** in order to monitor the lateral alignment of the eye relative to the main optical axis (A1), both reticles (11,12) are illuminated by means of an incoherent light beam and the eye is aligned laterally relative to the main optical axis (A1) in such a manner that the images of the two reticles seen by the eye (3) are superposed.

9. A method according to one of claims 1 to 4, **characterized in that** a converging lens (14) and two reticles (11,12) disposed on either side of the second lens (14) and centered on the optical axis of said lens (14) are used, and **in that** the longitudinal alignment of the eye on the main optical axis (A1) is monitored by illuminating both reticles (11,12) by means of an incoherent light beam, and the intensity of the return beam reflected by the eye is monitored.

10. A method according to claim 8 or claim 9, **characterized in that** the two reticles (11,12) are identical and positioned at a distance (2f') from the lens (14), where f' is the focal length of the lens (14).

11. A method according to claim 2 and according to one of claims 8 to 10, **characterized in that** one (11) of the two reticles is positioned at a distance (2f) from the correction lens (8), where $\underline{f}$ is the focal length of said lens.

12. A method according to any one of claims 1 to 11, **characterized in that** once the eye has been aligned longitudinally and laterally relative to the main optical axis (A1), the cornea is set into vibration by a blink of the eyelid or by a plurality of repeated blinks of the eyelid.

13. A detection device for using laser interferometry to detect at least one natural mode of vibration of an eye, said device comprising:

- a Fabry-Perot cavity having a main optical axis (A1), one of the two reflecting faces being formed by a reflecting element (M1) aligned on the optical axis (A1), and the other reflecting face being formed, when the device is in use, by the cornea (2) of the eye (3) in which it is desired to detect at least one natural mode of vibration;
- a laser source for emitting an incident laser beam (F) on the Fabry-Perot cavity; and
- an optoelectronic detector (7) for detecting the intensity (I) of longitudinal interference between the go and the return beams reflected along the main optical axis (A1) between the two reflecting faces (M1,2) of the Fabry-Perot cavity,

said device being **characterized in that** the laser source is positioned in such a manner that the incident laser beam (F) is aligned and centered on the main optical axis (A1) of the cavity.

14. A detection device according to claim 13, **characterized in that** it includes a converging correction lens (8) positioned between the two reflecting faces of the Fabry-Perot cavity, the lens being centered on the main optical axis (A1) and serving to reduce sensitivity to lateral misalignment of the eye.

15. A device according to claim 14, **characterized in that** the correction lens (8) possesses a short focal length (f) of less than 50 mm, and preferably of less than 30 mm.

16. A device according to claim 13, **characterized in that**, in order to monitor the longitudinal alignment of the eye along the main optical axis (A1) and/or the lateral alignment of the eye relative to the main optical axis (A1), use is made of a reticle (9,10) positioned on the main optical axis (A1) between the two reflecting faces of the Fabry-Perot cavity.

17. A device according to claims 14 and 16, **characterized in that** the reticle (9,10) is positioned between the reflecting element (M1) and the correction lens (8) in the focal plane of the correction lens.

18. A device according to claim 13, **characterized in that**, in order to monitor the longitudinal alignment of the eye on the main optical axis (A1) and/or the lateral alignment of the eye relative to the main optical axis (A1), the device includes a converging lens (14) and two reticles (11,12) disposed on either side of the second lens (14) and centered on the optical axis of said lens (14), and a light source enabling the two reticles (11,12) to be illuminated by means of an incoherent light beam.

19. A device according to claim 18, **characterized in that** the two reticles (11,12) are identical and are positioned at a distance (2f') from the lens (14), f' being the focal length of the lens (14).

20. A device according to claims 14 and 18, **characterized in that** one (11) of the two reticles is positioned at a distance (2f) from the correction lens (8), where f is the focal length of said lens.

21. A method of measuring the intraocular pressure of an eye by setting it into vibration and detecting at least one mode of vibration of the eye in accordance with the method specified in any one of claims 1 to 12.


**Patentansprüche**

1. Verfahren zur Erfassung wenigstens einer Eigenschwingungsmode eines Auges mittels Laserinterferometrie, wobei das Verfahren die Schritte umfaßt, die darin bestehen:

- das Auge (3) in bezug auf eine Erfassungsvorrichtung (1) derart zu positionieren, daß es mit dieser Vorrichtung eine Pérot-Fabry-Kavität mit der optischen Hauptachse (A1) bildet, die entlang der optischen Hauptachse (A1) zwei gegenüberliegende reflektive Flächen aufweist, wobei eine dieser beiden Flächen von einem festen reflektiven Element (M1) und die andere von der Hornhaut (2) des Auges (3) gebildet ist,
- in diese Kavität einen einfallenden Laserstrahl (F) einzuleiten, wobei die seitliche Ausrichtung und die Längsausrichtung der Hornhaut (2) in bezug auf die optische Hauptachse (A1) derart realisiert sind, daß zwischen den Hin- und Rücklaserstrahlen, die zwischen den beiden reflektiven Flächen der Kavität (M1, 2) reflektiert werden, longitudinale Interferenzen erhalten werden, und
- mittels eines optoelektronischen Detektors (7) die zeitliche Intensität (I) dieser Interferenzen zu erfassen,

wobei das Verfahren **dadurch gekennzeichnet ist, daß** der einfallende Laserstrahl (F) auf die optische Hauptachse (A1) der Pérot-Fabry-Kavität ausgerichtet und zu dieser zentrisch ist.

2. Verfahren nach Anspruch 1, **dadurch gekennzeichnet, daß** zwischen dem Auge (3) und dem reflektiven Element (M1) eine konvergente Korrekturlinse (8) angeordnet wird, die zur optischen Hauptachse (A1) zentrisch ist und ermöglicht, die Seitenabweichungsempfindlichkeit des Auges gegenüber der optischen Hauptachse (A1) zu verringern.

3. Verfahren nach Anspruch 2, **dadurch gekennzeichnet, daß** die Korrekturlinse (8) eine kurze Brennweite (f) von weniger als 50 mm und vorzugsweise von unter 30 mm aufweist.

4. Verfahren nach einem der Ansprüche 1 bis 4, **dadurch gekennzeichnet, daß** ein Sehzeichen (9) verwendet wird, das in der Pérot-Fabry-Kavität positioniert und zur optischen Hauptachse (A1) zentrisch ist, und daß das Auge in bezug auf die optische Hauptachse (A1) derart seitlich ausgerichtet wird, daß das mit dem Auge gesehene Sehzeichen nicht verformt wird.

5. Verfahren nach den Ansprüchen 2 und 4, **dadurch gekennzeichnet, daß** das Sehzeichen (9) zwischen dem reflektiven Element (M1) und der Korrekturlinse (8) in der Brennebene der Korrekturlinse (8) positioniert ist.

**6.** Verfahren nach einem der Ansprüche 1 bis 4, **dadurch gekennzeichnet, daß** ein Sehzeichen (10) verwendet wird, das in der Pérot-Fabry-Kavität positioniert und zur optischen Hauptachse (A1) zentrisch ist, und daß das Auge entlang der optischen Hauptachse (A1) derart längs ausgerichtet wird, daß dieses Sehzeichen (10) mit dem Auge sichtbar ist.

**7.** Verfahren nach den Ansprüchen 2 und 6, **dadurch gekennzeichnet, daß** das Sehzeichen (10) zwischen dem reflektiven Element (M1) und der Korrekturlinse (8) in der Brennebene der Korrekturlinse (8) positioniert ist.

**8.** Verfahren nach einem der Ansprüche 1 bis 4, **dadurch gekennzeichnet, daß** eine konvergente Linse (14) und zwei Sehzeichen (11, 12), die auf beiden Seiten der zweiten Linse (14) angeordnet und zur optischen Achse der Linse (14) zentrisch sind, verwendet werden, und daß zur Kontrolle der seitlichen Ausrichtung des Auges in bezug auf die optische Hauptachse (A1) die beiden Sehzeichen (11, 12) mittels eines inkohärenten Lichtstrahls beleuchtet werden, und das Auge in bezug auf die optische Hauptachse (A1) derart seitlich ausgerichtet wird, daß die Bilder der beiden mit dem Auge (3) gesehenen Sehzeichen übereinander liegen.

**9.** Verfahren nach einem der Ansprüche 1 bis 4, **dadurch gekennzeichnet, daß** eine konvergente Linse (14) und zwei Sehzeichen (11, 12), die auf beiden Seiten der zweiten Linse (14) angeordnet und zur optischen Achse der Linse (14) zentrisch sind, verwendet werden, und daß zur Kontrolle der Längsausrichtung des Auges entlang der optischen Hauptachse (A1) die beiden Sehzeichen (11, 12) mittels eines inkohärenten Lichtstrahls beleuchtet werden, und die Intensität des durch das Auge reflektierten Rückstrahls kontrolliert wird.

**10.** Verfahren nach Anspruch 8 oder 9, **dadurch gekennzeichnet, daß** die beiden Sehzeichen (11, 12) identisch sind und in einem Abstand (2f) von der Linse (14) angeordnet sind, wobei f die Brennweite der Linse (14) ist.

**11.** Verfahren nach Anspruch 2 und nach einem der Ansprüche 8 bis 10, **dadurch gekennzeichnet, daß** eines (11) der beiden Sehzeichen in einem Abstand (2f) von der Korrekturlinse (8) angeordnet ist, wobei f die Brennweite dieser Linse ist.

**12.** Verfahren nach einem der Ansprüche 1 bis 11, **dadurch gekennzeichnet, daß** sobald das Auge in bezug auf die optische Hauptachse (A1) längs und seitlich ausgerichtet ist, die Hornhaut durch ein Blinzeln oder mehrmaliges wiederholtes Blinzeln des Augenlides in Schwingung versetzt wird.

**13.** Vorrichtung zur Erfassung wenigstens einer Eigenschwingungsmode eines Auges mittels Laserinterferometrie, wobei die Vorrichtung folgendes umfaßt:

- eine Pérot-Fabry-Kavität mit der optischen Hauptachse (A1), wobei eine ihrer beiden reflektiven Flächen von einem auf die optische Achse (A1) ausgerichteten reflektiven Element (M1) und ihre andere reflektive Fläche während des Einsatzes der Vorrichtung von der Hornhaut (2) des Auges (3) gebildet ist, von dem wenigstens eine Eigenschwingungsmode erfaßt werden soll,
- eine Laserquelle, die das Aussenden eines auf die Pérot-Fabry-Kavität auftreffenden Laserstrahls (F) ermöglicht, und
- einen optoelektronischen Detektor (7) für die Erfassung der Intensität (I) der longitudinalen Interferenzen zwischen den Hin- und Rückstrahlen, die entlang der optischen Hauptachse (A1) zwischen den beiden reflektiven Flächen (M1, 2) der Pérot-Fabry-Kavität reflektiert werden,

wobei die Vorrichtung **dadurch gekennzeichnet ist, daß** die Laserquelle derart angeordnet ist, daß der einfallende Laserstrahl (F) auf die optische Hauptachse (A1) der Kavität ausgerichtet und zu dieser zentrisch ist.

**14.** Erfassungsvorrichtung nach Anspruch 13, **dadurch gekennzeichnet, daß** sie eine konvergente Korrekturlinse (8) aufweist, die zwischen den beiden reflektiven Flächen der Pérot-Fabry-Kavität angeordnet ist, zur optischen Hauptachse (A1) zentrisch ist und ermöglicht, die Seitenabweichungsempfindlichkeit des Auges zu verringern.

**15.** Vorrichtung nach Anspruch 14, **dadurch gekennzeichnet, daß** die Korrekturlinse (8) eine kurze Brennweite (f) von weniger als 50 mm und vorzugsweise von unter 30 mm aufweist.

**16.** Vorrichtung nach Anspruch 13, **dadurch gekennzeichnet, daß** zur Kontrolle der Längsausrichtung des Auges entlang der optischen Hauptachse (A1) und/oder der seitlichen Ausrichtung des Auges in bezug auf die optische Hauptachse (A1) ein Sehzeichen (9, 10) verwendet wird, das auf der optischen Hauptachse (A1) zwischen den beiden reflektiven Flächen der Pérot-Fabry-Kavität angeordnet ist.

17. Vorrichtung nach den Ansprüchen 14 und 16, **dadurch gekennzeichnet, daß** das Sehzeichen (9, 10) zwischen dem reflektiven Element (M1) und der Korrekturlinse (8) in der Brennebene der Korrekturlinse (8) positioniert ist.

18. Vorrichtung nach Anspruch 13, **dadurch gekennzeichnet, daß** sie für die Kontrolle der Längsausrichtung des Auges entlang der optischen Hauptachse (A1) und/oder der seitlichen Ausrichtung des Auges in bezug auf die optische Hauptachse (A1) eine konvergente Linse (14) und zwei Sehzeichen (11, 12), die auf beiden Seiten der zweiten Linse (14) angeordnet und zur optischen Achse der Linse (14) zentrisch sind, sowie eine Lichtquelle umfaßt, die ermöglicht, die beiden Sehzeichen (11, 12) mittels eines inkohärenten Lichtstrahls zu beleuchten.

19. Vorrichtung nach Anspruch 18, **dadurch gekennzeichnet, daß** die beiden Sehzeichen (11, 12) identisch sind und in einem Abstand (2f) von der Linse (14) angeordnet sind, wobei f die Brennweite der Linse (14) ist.

20. Vorrichtung nach den Ansprüchen 14 und 18, **dadurch gekennzeichnet, daß** das eine (11) der beiden Sehzeichen in einem Abstand (2f) von der Korrekturlinse (8) angeordnet ist, wobei f die Brennweite dieser Linse ist.

21. Verfahren zur Messung des intraokularen Drucks eines Auges durch In-Schwingung-versetzen und Erfassung wenigstens einer Schwingungsmode des Auges entsprechend dem Verfahren nach einem der Ansprüche 1 bis 12.

FIG.1

FIG.2

FIG.3

EP 1 335 663 B1

G

A2

F

L4

H

L2

H

L6

L5

L3

L1

A

H

B

H

C

I

D

E

H

A1

2a

3

1

FIG.15

10

9

FIG.4

FIG.5

# FIG.6

EP 1 335 663 B1

FIG.7

FIG.8

FIG.9

FIG.10

FIG.11

FIG.12

FIG.13

FIG.14

FIG.16

**EP 1 335 663 B1**

## RÉFÉRENCES CITÉES DANS LA DESCRIPTION

**Documents brevets cités dans la description**

- WO 9321820 A